(19) Europäisches Patentamt
European Patent Office
Office européen des brevets

(11) Publication number: **0 110 568 A1**

(12) **EUROPEAN PATENT APPLICATION**

(21) Application number: 83306559.2

(22) Date of filing: 27.10.83

(51) Int. Cl.³: A 61 K 7/16, A 61 K 7/22

(30) Priority: 29.10.82 US 437644
29.10.82 US 437645
16.08.83 US 523523
16.08.83 US 523521

(43) Date of publication of application: 13.06.84 Bulletin 84/24

(84) Designated Contracting States: AT BE CH DE FR IT LI NL SE

(71) Applicant: THE PROCTER & GAMBLE COMPANY, 301 East Sixth Street, Cincinnati Ohio 45201 (US)

(72) Inventor: Witt, Jonathan James, 2905 Banning Road No. 7, Cincinnati Ohio 45239 (US)
Inventor: Parran, John Joseph, Jr., 1155 Meredith Drive, Cincinnati Ohio 45231 (US)

(74) Representative: Brooks, Maxim Courtney et al, Procter & Gamble (NTC) Limited Whitley Road Longbenton, Newcastle-upon-Tyne NE12 9TS (GB)

(54) **Oral compositions.**

(57) Oral compositions such as toothpastes and mouthwashes containing an antimicrobial agent which is effective against plaque/gingivitis, a sufficient amount of a carboxylate compound to reduce the amount of staining caused by the antimicrobial and a water soluble magnesium salt are disclosed.

EP 0 110 568 A1

# ORAL COMPOSITIONS

The present invention relates to oral compositions containing antimicrobial agent effective against plaque and a sufficient amount of a carboxylate compound and optionally a water soluble magnesium salt to reduce the stain on teeth caused by the antimicrobial agent.

The use of antimicrobial agents to reduce plaque has been recognized for many years. The staining problems associated with such agents have also been recognized. Included among references disclosing antiplaque/antistain compositions are US-A-3,937,805, US-A-3,937,807, US-A-4,080,441, US-A-4,118,474, US-A-4,241,049, US-A-3,925,543 and US-A-4,256,731.

The use of magnesium salts in oral products generally has also been disclosed in the prior art. See for example US-A-4,217,342, US-A-4,259,316 and US-A-4,309,409.

While the prior art discloses the usefulness of antimicrobials and attempts to reduce the stain caused by the antimicrobials, completely satisfactory answers have not been obtained.

Plaque is believed to be the result of bacteria in the mouth which react with nutrients in saliva to form a deposit on tooth surfaces. Antimicrobials have been shown to be able to interrupt this cycle and thereby reduce the level of plaque formed. However, the antimicrobials are believed to induce mineralization of plaque, thereby trapping coloured entities.

It has now been found that by combining a carboxylate compound with the antimicrobial, a reduction in staining can be achieved while still providing the therapeutic efficacy of the compositions and the decrease in staining can be extended by the addition of a water soluble magnesium salt.

It is therefore an object of the present invention to formulate effective antiplaque/antigingivitis compositions. By reducing plaque, caries, and/or calculus may also be reduced.

It is a further object of the present invention to formulate antiplaque/antigingivitis compositions which have reduced staining.

It is still a further object of the present invention to provide a method for reducing plaque/gingivitis.

These and other objects will become more apparent from the detailed description which follows. All percentages and ratios herein are by weight unless otherwise specified.

The present invention relates to oral compositions which provide antiplaque/antigingivitis benefits while causing less stain, comprising:

a) a safe and effective amount of an antimicrobial;

b) a carboxylic acid having a $pK_a$ in the range of about 3 to about 6 or a water soluble salt thereof or mixtures of said salts and acids, wherein both the acid and the counter ion of the salt are compatible with the antimicrobial;

c) optionally, a water soluble magnesium salt; and

d) a pharmaceutically acceptable carrier;

wherein the molar ratio of the carboxylic acid or salt thereof to the antimicrobial is at least 70:1, the molar ratio of magnesium, if present, to the antimicrobial is at least 10:1 and the pH of the composition is from about 3 to about 6, preferably from about 4 to about 6, and/or has such a pH when dissolved in water or saliva.

The compositions of this invention employ an antimicrobial in combination with a carboxylic acid or water soluble salt thereof and, optionally and preferably, a water soluble magnesium salt. These and other components will be described in detail hereinafter.

By "safe and effective amount of an antimicrobial," as used herein, means sufficient compound to reduce plaque while being safe to the hard and soft tissues of the oral cavity.

By "compatible with the antimicrobial" as used herein is meant that the carboxylic acid or the counter ion of a salt thereof or any other material present will not form an insoluble precipitate with the antimicrobial.

By the term "comprising," as used herein, is meant that various additional components can be conjointly employed in the compositions of this invention as long as the antimicrobial and the carboxylic acid or salt thereof can perform their intended functions.

By the term "carrier," as used herein, is meant a suitable vehicle which is pharmaceutically acceptable and can be used to apply the present compositions in the oral cavity.

By the term "water soluble," as used herein, is meant having a solubility sufficient to provide the quantities required herein (at least about 0.023 g/100 g. at 25°C).

Antimicrobial

The antimicrobials used in the compositions of the present invention can be any of a wide variety of materials which may function in many different ways to reduce plaque/gingivitis. Included among these materials are cationic antimicrobial agents such as quaternary ammonium (e.g. cetyl pyridinium chloride) compounds and substituted guanidines such as chlorhexidine and the corresponding compound alexidine; phenolics (e.g. salicylanilide); tertiary amines. (e.g. hexetidine); peroxides such as hydrogen peroxide; and materials such as antibiotics (e.g. penicillin). The preferred materials are the cationic antimicrobials, particularly the substituted guanidines.

Antimicrobial quaternary ammonium compounds include those in which one or two of the substituents on the quaternary nitrogen has a carbon chain length (typically alkyl group) of some 8 to 20, typically 10 to 18 carbon atoms while the remaining substituents (typically alkyl or benzyl group) have a lower number of carbon atoms, such as 1 to 7 carbon atoms, typically methyl or ethyl groups. Dodecyl trimethyl ammonium bromide, dodecyl dimethyl (2-phenoxyethyl) ammonium bromide, benzyl dimethylstearyl ammonium chloride, cetyl pyridinium chloride, quaternized 5-amino-1,3-bis(2-ethyl-hexyl)-5-methyl hexa hydropyrimidine and benzethonium chloride are exemplary of typical quaternary ammonium antibacterial agents. Cetyl pyridinium chloride is a preferred quaternary ammonium compound.

The substituted guanidines of this invention include bisbiguanide compounds having the generic formula

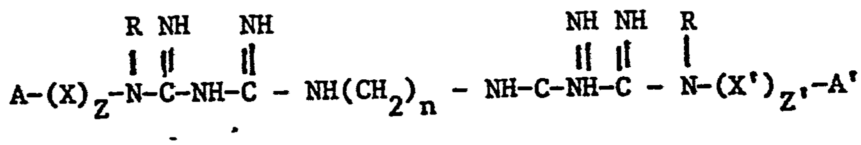

wherein A and A' each represent either (1) a phenyl radical which optionally is substituted by an alkyl or alkoxy group containing from 1 to about 4 carbon atoms, a nitro group, or a halogen atoms; (2) an alkyl group containing from 1 to about 12 carbon atoms; or (3) alicyclic groups containing from 4 to about 12 carbon atoms; wherein X and X' each represent an alkylene radical containing from 1 to 3 carbon atoms; wherein Z and Z' each can be either 0 or 1; wherein R and R' each represent either hydrogen, an alkyl radical containing from 1 to about 12 carbon atoms, or an aralkyl radical containing from 7 to about 12 carbon atoms; wherein n is an integer from 2 to 12 inclusive; wherein the polymethylene chain $(CH_2)_n$ may optionally be interrupted by oxygen or sulfur atoms, aromatic nuclei, etc. The water soluble salts of the above compounds are preferred for use herein. Suitable water soluble salts include the acetate, the fluoride, and especially the gluconate salt. The preferred substituted guanidine is chlorhexidine - [1,6-di(-$N^5$-p-chlorophenyl-N-diguanido)hexane].

The above compounds are effective anti-plaque/antigingivitis agents However, when compositions containing these compounds are used continuously in a program of oral hygiene, a rather offensive brown stain forms on the oral surfaces which is resistant to removal. This stain problem prevents compositions containing these compounds from being accepted by the consumer when used at levels sufficient to deliver a significant anti-plaque benefit.

The antimicrobial is used in an amount that is effective while yet being safe to use in the oral cavity. Such amounts are easily determined by the skilled artisan and are generally from about 0.001% to about 10%, preferably from about 0.01% to about 2%, most preferably from about 0.05% to about 1.0%.

Many more acceptable antimicrobials and their properties are found in Franklin, T.J. and Snow, G.A., Biochemistry of Antimicrobial Actions, Chapman and Hall, London and John Wiley & Sons, N.Y. 2nd. Ed.(1975), incorporated herein by reference.

Carboxylic Acid or Water Soluble Salt Thereof

The carboxylic acids suitable for use in the present compositions are those water soluble acids which have a $pK_a$ of from about 3 to about 6, preferably is from about 4.5 to about 5.5; and are compatible with the antimicrobial. The carboxylic acid and/or its water soluble salt is present in a molar ratio of at least 70:1, preferably from about 75:1 to about 750:1. The water soluble salts of such acids found most useful are those wherein the counter ions are sodium, lithium, potassium, magnesium and zinc.

Suitable acids include formic, gluconic, lactic, acetic, propionic, butyric, valeric, caproic, caprylic phenylacetic, p-hydroxybenzoic, m-aminobenzoic, malic, adipic, isobutyric and hydroxybutyric. These and others are found in Morrison R. T. Boyd, R. N. Organic Chemistry, Allen & Bacon, Inc., Boston

MA, 3rd Ed. (1973) incorporated herein by reference. The carboxylic acid may be a mono, di, tri or polycarboxylic acid. It may also be substituted, saturated or unsaturated. The substituents should also be compatible with the antimicrobial. The preferred acids include acetic acid, gluconic acid, adipic acid and mixtures thereof.

Pharmaceutically Acceptable Carrier

The carrier for the antimicrobial, the carboxylate compound and the preferred magnesium salt can be any vehicle suitable for use in the oral cavity. Such carriers include the usual components of mouthwashes, toothpastes, tooth powders, prophylaxis pastes, and the like and is more fully described hereinafter.

One embodiment of the present invention is a dentifrice, especially a toothpaste. Dentifrices preferably contain from about 0.1% to 2.0% by weight of the antimicrobial component. Dentifrices also contain an abrasive polishing material and typically also contain sudsing agents, flavoring agents and sweetening agents. Toothpaste compositions additionally contain binders, humectants and water.

The dentifrice abrasive, generally has a particle size of from about 0.1 to about 10 microns in diameter and can be any abrasive polishing material which does not excessively abrade tooth dentin. These include, for example, silica, both precipitated and gels, calcium carbonate, dicalcium orthophosphate dihydrate, calcium pyrophosphate, calcium polymetaphosphate and insoluble sodium polymetaphosphate. Preferably, however, the abrasive is one which has a high degree of compatibility with the bisbiguanides. These include, for example silica xerogels such as those described in U.S. Patent 3,538,230 to Pader et al, issued November 3, 1970; hydrofluoric acid-treated amorphous silica abrasives such as those disclosed in U.S. Patent 3,862,307 to DiGiulio, issued January 21, 1975; mineral abrasives coated with cationic polymers such as those disclosed by J. J. Benedict in

BAD ORIGINAL

U.S. Patent 4,157,387, issued June 5, 1979; and condensation products of urea and formaldehyde such as those disclosed in Cooley et al, in U.S. Patent 3,070,510, issued December 25, 1972. All of these patents are incorporated by reference.

The total amount of abrasive materials in the dentifrice embodiments of this invention can range from about 0.5% to about 95% by weight of the dentifrice. Preferably toothpastes contain from about 6% to about 60% by weight and toothpowders contain from about 20% to about 95% by weight abrasives.

Dentifrice compositions can also contain emulsifying agents. Suitable emulsifying agents are those which are reasonably stable and foam throughout a wide pH range, and which will not react with the bis-biguanide compound, i.e., non-soap nonionic, cationic, zwitterionic and amphoteric organic synthetic detergents. Many of these suitable surfactants are disclosed by Gieske et al in U.S. Patent 4,051,234, September 27, 1977, incorporated herein by reference.

It is common to have a water-soluble fluoride compound present in dentifrices in an amount sufficient to give a fluoride concentration of from about 0.0025% to about 5.0% by weight, preferably from about 0.005% to about 2.0% by weight, to provide additional anticaries effectiveness. Preferred fluorides are sodium fluoride, stannous fluoride, indium fluoride, and sodium monofluorophosphate. Norris et al, U.S. Patent 2,946,725, issued July 26, 1960 and Widder et al, U.S. Patent 3,678,154, issued July 18, 1972.

In preparing toothpastes, it is necessary to add some thickening material to provide a desirable consistency. Preferred thickening agents are carboxyvinyl polymers, hydroxyethyl cellulose and water soluble salts of cellulose ethers such as sodium carboxymethyl cellulose and sodium carboxymethyl hydroxyethyl cellulose. Natural gums such as gum karaya, gum arabic, and gum tragacanth can also be used. Colloidal magnesium aluminum silicate or finely divided silica can be used as part of the thickening agent to further improve texture. Thickening agents in an amount from 0.5% to 5.0% by weight of the total composition can be used.

It is also desirable to include some humectant material in a toothpaste to keep it from hardening. Suitable humectants include glycerin, sorbitol, and other edible polyhydric alcohols. The humectant can comprise up to about 65% by weight of the toothpaste composition.

Flavoring agents can also be added to dentifrice compositions. Suitable flavoring agents include oil of wintergreen, oil of peppermint, oil of spearmint, oil of sassafras, and oil of clove. Sweetening agents which can be used include aspartame, acesulfame, saccharin, dextrose, levulose and sodium cyclamate. Flavoring agents are generally used in dentifrices at levels of from about 0.05% to about 2% by weight.

A preferred embodiment of the instant invention is a mouthwash composition. Conventional mouthwash composition components can comprise the carrier for the antimicrobial and carboxylate of the present invention. Mouthwashes generally comprise about 20:1 to about 2:1 of a water/ethyl alcohol solution and preferably other ingredients such as flavor, sweeteners, humectants and sudsing agents such as those mentioned above for dentifrices. The humectants, such as glycerin and sorbitol give a moist feel to the mouth. Generally, on a weight basis the mouthwashes of the invention comprise 5% to 60% (preferably 10% to 25%) ethyl alcohol, 0% to 20% (preferably 5% to 20%) of a humectant, 0% to 2% (preferably 0.01% to 0.15%) emulsifying agent, 0% to 0.5% (preferably 0.005% to 0.06%) sweetening agent such as saccharin, 0% to 0.3% (preferably 0.03% to 0.3%) flavoring agent, and the balance water. The amount of antimicrobial agent in mouthwashes is typically from about 0.01% to about 0.2% by weight.

Water Soluble Magnesium Salt

The compositions herein preferably contain a water soluble magnesium salt whose counter-ion is compatible with the antimicrobial. The salts can be any of a wide variety including the acetate, gluconate and adipate. The magnesium salt is present in an amount such that magnesium is present at a

BAD ORIGINAL

molar ratio to the antimicrobial of at least 10:1, preferably 70:1 to about 150:1, most preferably about 100:1. Mixtures of magnesium salts may also be used. The magnesium compound serves to extend the antistain benefit delivered by the carboxylate compound.

## METHOD OF MANUFACTURE

The carrier compositions of the present invention can be made using methods which are common in the oral products area. A typical mouthwash making procedure is shown in the examples.

## COMPOSITION USE

The present invention in its method aspect involves applying to the oral cavity safe and effective amounts of the antimicrobial and the carboxylate compound. Generally an amount of at least about 0.001g. of the antimicrobial is effective. The two agents can be applied from one composition or sequentially from two.

The following examples further describe and demonstrate preferred embodiments within the scope of the present invention. The examples are given solely for illustration and are not to be construed as limitations of this invention as many variations thereof are possible without departing from the spirit and scope thereof.

## EXAMPLES I - VI

The following are mouthwash compositions representative of the present invention.

| Component/ Composition | I | II | III | IV | V | VI |
|---|---|---|---|---|---|---|
| Acetic Acid | 0.62% | 1.60% | 1.60% | 0.51% | 0.51% | 1.60% |
| Sodium Hydroxide(50%) | 0.23 | 1.35 | 1.20 | - | - | 1.35 |
| Chlorhexidine Digluconate | 0.12 | 0.12 | .075 | 0.12 | 0.20 | - |
| Cetylpyridinium Chloride | - | - | - | - | - | .075 |
| Magnesium Acetate Tetrahydrate | - | - | - | 1.95 | 1.95 | - |
| Ethyl Alcohol (190 proof) | 10% → | | | | | |
| Glycerin | 8% → | | | | | |
| Flavor | 0.03-0.08% → | | | | | |
| Sodium Saccharin | 0.01% → | | | | | |
| Polyethylene Glycol 40 Sorbitan diisostearate | .075 | .075 | .105 | .075 | .075 | 0.105 |
| Water | q.s.100 → | | | | | |
| pH | 4.5 | 5.0 | 5.0 | 5.0 | 5.0 | 5.0 |

The above compositions can be prepared by mixing the ethyl alcohol, the sorbitan diisostearate, the flavor and the glycerin together for about 10 minutes. Water is then added with mixing followed by the acetic acid and sodium hydroxide or magnesium acetate and acetic acid. This is followed by the addition of sweetener and then the antimicrobial.

BAD ORIGINAL

## EXAMPLE VII

The ability of a carboxylic acid of this invention, acetic acid, to reduce stain caused by an antimicrobial, chlorhexidine digluconate, was demonstrated in an in-vivo study.

The products tested were as follows:

| Component/Composition | A | B |
|---|---|---|
| Chlorhexidine Digluconate | 0.120 | 0.120 |
| Ethanol (190 proof) | 10.000 | 10.000 |
| Glycerin | 8.000 | 8.000 |
| PEG-40 Sorbitan Diisostearate | 0.075 | 0.075 |
| Sodium Saccharin | 0.010 | 0.010 |
| Flavor | 0.050 | 0.050 |
| Sodium hydroxide (50% Aqueous Solution) | - | 1.350 |
| Acetic Acid | - | 1.600 |
| Water | 81.745 | 78.795 |
| | 100.000 | 100.000 |
| pH | 6.5 | 5.0 |

### Test Design

Eighteen subjects in the age range of 22 - 26 years were used in this study. The subjects were given a thorough prophylaxis and were asked to perform optimal oral hygiene for a period of two weeks, including toothbrushing, dental floss and interdental wooden sticks for interproximal cleaning. This period of oral hygiene was supervised by a registered dental hygienist. The participants were rescreened for use of oral antibiotics and any oral lesions. At this point, the subjects were randomly divided into two groups and were asked to abolish all oral hygiene procedures for a period of 21 days. During this time the

BAD ORIGINAL

subjects rinsed with 15 ml. of one of the above mouthwashes for 30 seconds two times each day, with the exception of Saturdays and Sundays when subjects rinsed only once. All rinses were supervised by a registered dental hygienist.

After the 21 day period the subjects' teeth were graded for plaque (using the method of Silness and Loe) and stain (using the method of Lang and Raber). The mean scores for plaque and stain of each group are shown below.

| | Gingivitis[1] | Plaque[2] | Stain[3] |
|---|---|---|---|
| Subjects Using A | 0.26 | 1.27 | 2.06 |
| Subjects Using B | 0.25 | 1.58 | 1.21 |

1 Lower score indicates less gingivitis. Difference not significant at $\alpha=0.05$.

2 Lower score indicates less plaque. Difference not significant at $\alpha=0.05$.

3 Lower score indicates less stain. Difference is significant at $\alpha=0.05$.

CLAIMS

1. An oral composition effective in inhibiting plaque/gingivitis and reducing stain caused by antimicrobial compounds characterized by:

A. a safe and effective amount of an antimicrobial;

B. a water soluble carboxylic acid having a $pK_a$ in the range of from 3 to 6 or a water soluble salt thereof or mixtures of the acids and salts thereof;

C. optionally, a water soluble magnesium salt; and

D. a pharmaceutically acceptable carrier;

wherein both the carboxylic acid and the counter ions of the water soluble carboxylate salt and magnesium salt are compatible with the antimicrobial, the molar ratio of carboxylic acid and/or water soluble salt to antimicrobial is at least 70:1, the molar ratio of magnesium, if present, to antimicrobial is at least 10:1 and wherein the composition has a pH of from 3 to 6 and/or has such a pH when dissolved in water or saliva.

2. An oral composition according to Claim 1 characterized by from 0.01% to 10% by weight of antimicrobial and wherein the composition has a pH of from 4 to 6, and/or has such a pH when dissolved in water or saliva.

3. An oral composition according to Claim 1 or 2 characterized in that the antimicrobial is selected from cationic compounds, tertiary amines, phenolics, peroxides and antibiotics.

4. An oral composition according to Claim 3 characterized in that the antimicrobial is selected from quaternary ammonium compounds and substituted guanidines.

5. An oral composition according to Claim 4 characterized in that the antimicrobial is selected from chlorhexidine, alexidine and water soluble salts thereof.

6. An oral composition according to any of Claims 1 to 5 characterized in that the carboxylic acid is selected from acetic acid, gluconic acid, adipic acid and mixtures thereof.

7. An oral composition according to any of Claims 1 to 6 characterized in that the water soluble magnesium salt is selected from magnesium acetate, magnesium glyconate, magnesium adipate and mixtures thereof.

8. An oral composition according to any of Claims 1 to 7 characterized in that the antimicrobial is a water soluble salt of chlorhexidine selected from acetate, gluconate and fluoride salts.

9. An oral composition according to any of Claims 1 to 8 characterized in that the carboxylic acid is acetic acid present in a molar ratio to the antimicrobial of from 75:1 to 750:1.

10. An oral composition according to any of Claims 1 to 9 characterized in that the molar ratio of magnesium to antimicrobial is from 70:1 to 150:1.

11. An oral composition according to any of Claims 1 to 10 in the form of a mouthwash which composition additionally contains water and alcohol or in the form of a dentifrice which composition additionally contains an abrasive.

0110568

Application number

European Patent Office

# EUROPEAN SEARCH REPORT

EP 83 30 6559

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US-A-3 629 468 (ANDERSEN) * claims; column 1, lines 47-60; column 3, lines 15-33; example 1 * | 1-4,6 | A 61 K 7/16<br>A 61 K 7/22 |
| X | EP-A-0 004 719 (BEECHAM INC.) * claims * | 1-5,8, 11 | |
| X | US-A-4 022 880 (VINSON et al.) * example 11; column 14; example 24, column 17; claims * | 1,3-6, 8,11 | |
| D,Y | US-A-4 217 342 (GAFFAR et al.) * claims; column 10, line 59 - column 11, line 10 * | 1 | |

TECHNICAL FIELDS SEARCHED (Int. Cl. 3)

A 61 K 7/00

The present search report has been drawn up for all claims

| Place of search | Date of completion of the search | Examiner |
|---|---|---|
| THE HAGUE | 16-02-1984 | WILLEKENS G.E.J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document